# EUROPEAN PATENT APPLICATION

(11) **EP 4 767 964 A1**
(43) Date of publication of application: **01.07.2026**
(21) Application number: 25217161.6
(22) Date of filing: 20.11.2025
(51) Int. Cl.: A61B 18/14

(54) **ELECTROPHYSIOLOGY CATHETER SYSTEM WITH COMPOSITE ELECTRODE BASED ON CONTACT FORCE AND IMPEDANCE SENSING FOR IRREVERSIBLE-ELECTROPORATION (IRE) AND RELATED METHODS**

(30) Priority: 31.12.2024 US 202419006985
(71) Applicant: Biosense Webster (Israel) Ltd., 2066717 Yokneam (IL)
(72) Inventor: GOVARI, Assaf, 2066717 Yokneam (IL); ALTMANN, Andres C., 2066717 Yokneam (IL); BEECKLER, Christopher Thomas, Irvine, 92618 (US)
(74) Representative: Carpmaels & Ransford LLP

(57) **Abstract**

A catheter system for use with a catheter with a distal assembly supporting a plurality of electrodes adapted to sense electrical signal in heart tissue and a force sensor configured to generate first contact signals representative of a contact force acting on the distal assembly. The system includes an ablation power generator configured to energize the plurality of electrodes, an impedance circuitry configured to generate second contact signals representative of the electrodes in contact with tissue, a processor configured to receive the first and second contact signals and generate switch signals in response thereto, and a switch circuitry configured to connect in response to the switch signals solely selected electrodes in contact with tissue to the ablation power generator in forming one or more composite electrodes.

## Description

### FIELD OF INVENTION

The present invention relates generally to diagnostic and ablation catheters, and particularly diagnostic and ablation catheters configured for irreversible electroporation (IRE) of cardiac tissue in unipolar and bipolar mode.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals may be located in tissue of an atria or a ventricle. Unwanted signals may be conducted elsewhere through heart tissue where they can initiate or continue arrhythmia.

Procedures for treating arrhythmia include surgically disrupting the origin of the signals causing the arrhythmia, as well as disrupting the conducting pathway for such signals. By mapping the electrical properties of the endocardium and the heart volume, and selectively ablating cardiac tissue by application of energy, it may be possible to cease or modify the propagation of unwanted electrical signals from one portion of the heart to another. The ablation process may destroy the unwanted electrical pathways by formation of non-conducting regions of tissue.

In this two-step procedure, which includes mapping followed by ablation, electrical activity at points in the heart may be sensed and measured by advancing a first or mapping catheter containing one or more electrical sensors into the heart and acquiring data at multiple points. These data may then be utilized to select the target areas at which ablation is to be performed by a second or ablation catheter.

During ablation, RF current is applied to a first electrode of the ablation catheter and current flows through the media that surrounds it, i.e., blood and tissue, toward a second electrode which may be another electrode on the catheter or an external skin patch reference electrode. The distribution of current depends on the amount of electrode surface in contact with the tissue as compared to blood, which has a higher conductivity than the tissue. Heating of tissue occurs due to its electrical resistance. The tissue is heated sufficiently to cause cellular destruction in the target tissue resulting in formation of a lesion which is electrically non-conductive. The lesion may be formed in tissue contacting the electrode or in adjacent tissue. During this process, heating of the electrode also occurs as a result of conduction from the heated tissue to the electrode itself.

Using a multi-electrode catheter for irreversible electroporation (IRE) has been previously proposed in patent literature. For example, PCT International Publication WO 2018/191149 describes electroporation systems and methods of energizing a catheter for delivering electroporation. A catheter for delivery electroporation includes a distal section and an electrode assembly. The distal section is configured to be positioned in a vein within a body. The vein defines a central axis. The electrode assembly is coupled to the distal section and includes a structure and a plurality of electrodes distributed thereabout. The structure is configured to at least partially contact the vein. Each of the electrodes is configured to be selectively energized to form a circumferential ring of energized electrodes that is concentric with the central axis of the vein. In an embodiment, each electrode is individually wired such that it can be selectively paired or combined with any other electrode to act as a bipolar or a multi-polar electrode.

As another example, U.S. Patent No. 8,295,902 describes a tissue electrode assembly that includes a membrane configured to form an expandable, conformable body that is deployable in a patient. The assembly further includes a flexible circuit positioned on a surface of the membrane. An electrically-conductive electrode covers at least a portion of the flexible circuit and a portion of the surface of the membrane not covered by the flexible circuit, wherein the electrically-conductive electrode is foldable upon itself with the membrane to a delivery conformation having a diameter suitable for minimally-invasive delivery of the assembly to the patient. In an embodiment, a pattern of multiple electrodes deposited on the membrane can collectively create a large electrode array of energy-transmitting elements.

U.S. Application Serial No. 18/159,288, filed January 25, 2023, titled Electrode Designs for Catheters (hereinafter "the '288 application"), discloses technology that includes a catheter comprising an elongated deflectable element extending along a longitudinal axis from a proximal end to a distal end, a position electrode attached to the elongated deflectable element proximate the distal end and configured for impedance-based position tracking, and a covering at least partially enclosing the position electrode. The covering can comprise a plurality of apertures such that a portion of a conductive surface of the position electrode is exposed through each aperture of the plurality of apertures. The entire content of the '288 application is incorporated herein by reference, and attached hereto as Appendix A.

Applicants recognized there is a need to provide a catheter with electrodes configured for both diagnostic mapping and therapeutic ablation, where the electrodes can sense tissue electrical activity and ablate, with lower impedance and greater electrical conductivity, while operational in a variety of modalities including unipolar and bipolar configurations and biphasic voltage pulsation in pulsed field ablation (PFA) to cause irreversible tissue electroporation (IRE).

### SUMMARY OF THE DISCLOSURE

Embodiments described herein are directed to an electrophysiology system using a catheter with an end effector carrying multiple electrodes configured for both diagnostic mapping and therapeutic ablation, where the electrodes can sense tissue electrical activity and ablate with high voltage, lower impedance and greater electrical conductivity, while operational in a variety of modalities including unipolar and bipolar configurations and biphasic voltage pulsation, all via a composite electrode comprising solely of selected electrodes electrically connected on the basis contact force sensing and impedance sensing that identifies electrodes that are in contact with tissue, such that such connected electrodes can withstand high voltage in pulsed field ablation (PFA) to cause apoptosis .

In some embodiments, a catheter system comprises a catheter, an ablation power generator, an impedance circuitry, a processor and a switch circuitry. The catheter includes a basket assembly with a plurality of electrode configured to receive electrical signals from cardiac tissue and a contact force sensor, the contact force sensor configured to generate first contact signals representative of a contact force acting on the basket assembly. The ablation power generator is configured to energize one or more of the plurality of electrodes to deliver energy into the cardiac tissue. The impedance circuitry is configured to send an electrical current to each electrode and generate second contact signals for each electrode representative of contact between the electrode and heart tissue. The processor is configured to receive the first and second contact signals and identify selected electrodes in contact with tissue and generate switch signals in response to the first and second contact signals. The switch circuitry is configured to electrically connect in response to the switch signals solely the selected electrodes to the ablation power generator in forming a composite electrode.

In some embodiments, the switch circuitry is configured to electrically connect the selected electrodes for bipolar ablation.

In some embodiments, the switch circuitry is configured to electrically connect the selected electrodes for unipolar ablation.

In some embodiments, the basket assembly includes a shaft defining a longitudinal axis, a plurality of strips converge at their distal and proximal ends and an elongated pusher extending through the shaft and longitudinally movable relative to the shaft, the elongated pusher having a distal end connected to the distal ends of the strips such that proximal movement of the elongated pusher relative to the shaft along the longitudinal axis bows the strips outwardly in expanding the basket assembly.

In some embodiments, the catheter includes a position sensor housed in the pusher.

In some embodiments, distal movement of the elongated pusher relative to the shaft along the longitudinal axis after proximal movement collapses the basket assembly.

In some embodiments, the contact force sensor is housed in the shaft proximally of a distal end of the shaft.

In some embodiments, the contact force sensor includes a first end, a second end and a deformable spring therebetween.

In some embodiments, the contact force sensor further includes a magnetic field transmitter housed in the first end and a magnetic field receiver housed in the second end, the magnetic field receiver configured to be responsive to the magnetic field transmitter in generating displacement signals indicative of a displacement in spatial relationship between the magnetic field transmitter and the magnetic field sensor.

In some embodiments, the displacement signals are indicative of a distance and an angular direction of the displacement.

In some embodiments, each of the strips includes a flexible polymer circuit strip and an elongated resilient support element.

In some embodiments, each flexible polymer circuit strip includes multiple electrodes aligned along a length of the strip.

In some embodiments, the ablation power generator is configured to provide ablation energy to the electrodes for pulsed field ablation (PFA) to cause apoptosis in the heart tissue.

In some embodiments, the ablation voltage ranges between about 1kV to 3kV.

In some embodiments, the catheter includes a reference electrode.

In some embodiments, the reference electrode is mounted circumferentially on the pusher as a ring electrode.

In some embodiments, the system further comprises a user input device configured to command the catheter system to operate in a tissue sensing mode or an ablation mode.

In some embodiments, the user input device includes a hand-operable device.

In some embodiments, the user input device includes a foot-operable device.

In some embodiments, the switch circuitry includes a first switch configured to connect a respective electrode to the ablation power generator, a second switch configured to connect the respective electrode to the processing unit, and a third switch is configured as a float.

In some embodiments, the processing unit is configured to actuate the switch circuitry to connect the respective electrode to the ablation power generator via the first switch in response to at least one of a first contact signal and a second contact signal.

In some embodiments, the respective electrode is configured as an active electrode.

In some embodiments, the processing unit is configured to actuate the switch circuitry to connect the respective electrode to the processing unit via the second switch in response to at least one of a first contact signal and a second contact signal.

In some embodiments, the respective electrode is configured as a return electrode.

In some embodiments, the processing unit is configured to actuate the switch circuitry to connect the respective electrode to the third switch in the absence of a contact signal.

In some embodiments, a catheter system comprises a catheter, an ablation power generator, a processor and a switch circuitry. The catheter includes a plurality of electrodes and a plurality of force sensors, each force sensor configured to generate a respective contact signal representative of a contact force acting on a respective electrode. The ablation power generator is configured to energize the plurality of electrodes. The processor is configured to receive each respective contact signal and in response thereto generate a respective switch signal for each electrode. The switch circuitry is configured to electrically connect in response to each respective switch signal solely selected electrodes in contact with tissue to the ablation power generator in forming a composite electrode.

In some embodiments, the system includes a user input device configured to actuate the ablation power generator.

In some embodiments, the processor is configured to actuate the ablation power generator in a biphasic mode.

In some embodiments, the processor is configured to actuate the switch circuitry between a unipolar mode and a bipolar mode.

In some embodiments, the processing unit is configured to actuate the switch circuitry between a tissue sensing mode and an ablation mode.

In some embodiments, the processing unit is configured to actuate the switch circuitry in response to a control signal from the user input device.

In some embodiments, the catheter further comprises a reference electrode.

In some embodiments, the reference electrode is configured to avoid tissue contact.

In some embodiments, the switch circuitry includes a first switch configured to connect a respective electrode to the ablation power generator, a second switch configured to connect the respective electrode to the processing unit, and a third switch as a float.

In some embodiments, the processing unit is configured to actuate the switch circuitry to connect the respective electrode to the ablation power generator via the first switch in response to the contact signal.

In some embodiments, the respective electrode is configured as an active electrode.

In some embodiments, the processing unit is configured to actuate the switch circuitry to connect the respective electrode to the processing unit via the second switch in response to the contact signal.

In some embodiments, the respective electrode configured as a return electrode.

In some embodiments, the processing unit is configured to actuate the switch circuitry to connect the respective electrode to the third switch in the absence of the contact signal.

In some embodiments, the catheter includes an expandable distal assembly with spines, the electrodes disposed on different spines.

In some embodiments, the distal assembly is configured as a 3D form with an interior and a reference electrode is situated in the interior of the distal assembly.

In some embodiments, the catheter includes a shaft, the distal assembly extending from a distal end of the shaft, the distal assembly including a strut extending from the distal end of the shaft to an electrode, the struct configured to deform in response to a contact force acting on the electrode.

In some embodiments, a force sensor is affixed to the strut and configured to generate a contact signal in response to deformation of the strut.

### BRIEF DESCRIPTION OF THE DRAWINGS

These and other features and advantages of the present invention will be better understood by reference to the following detailed description when considered in conjunction with the accompanying drawings. It is understood that selected structures and features have not been shown in certain drawings so as to provide better viewing of the remaining structures and features.
FIG. 1 is a pictorial illustration of an electrophysiology system for sensing electrical activity in and performing ablation on heart tissue, in accordance with an embodiment of the invention.
FIG. 2A is a perspective view of a catheter for use with the system of FIG. 1, in accordance with an embodiment of the invention.
FIG. 2B is a side elevational view of a contact force sensor of FIG. 2A, in accordance with an embodiment of the invention.
FIG. 3 is a perspective view of a distal electrode assembly of FIG. 2A, in accordance with an embodiment of the invention.
FIG. 4A is a side cross-sectional view of a distal portion of the distal electrode assembly of FIG. 3, in accordance with an embodiment of the invention.
FIG. 4B is a side cross-sectional view of a proximal portion of the distal electrode assembly of FIG. 3, in accordance with an embodiment of the invention.
FIG. 5A and FIG. 5B are schematic views of the distal electrode assembly of FIG. 3, in an expanded configuration and a collapsed configuration, respectively, in accordance with an embodiment of the invention.
FIG. 6 is a block diagram of the system of FIG. 1, in accordance with an embodiment of the invention.
FIG. 7 is a flowchart illustrating a method 300 of diagnosing and treating heart arrhythmia, in accordance with an embodiment of the present invention.
FIG. 8 is a perspective view of a distal electrode assembly in an expanded configuration, in accordance with another embodiment of the invention.
FIG. 9 is a perspective view of a distal electrode assembly in an expanded configuration, in accordance with yet another embodiment of the invention.
FIG. 10 is a block diagram of a system configured for use with the distal electrode assembly of FIG. 9.
FIG. 11 is a block diagram of the system of FIG. 10 in tissue sensing mode, in accordance with one embodiment of the present invention.
FIG. 12 is a block diagram of the system of FIG. 10 in unipolar ablation mode, in accordance with one embodiment of the present invention.
FIG. 13 is a block diagram of the system of FIG. 10 in bipolar ablation mode, in accordance with one embodiment of the present invention.

### DETAILED DESCRIPTION FOR MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g., "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

Example end effectors are illustrated and disclosed herein which are generally planar and include multiple electrodes that can be configured for mapping and/or ablation. The end effectors can be joined to a shaft with additional catheter components to form a mapping and/or ablation catheter through processes disclosed herein and processes similar to those known by a person skilled in the pertinent art. The example end effectors illustrated herein include variations and features that are combinable to form additional end effector designs as understood by a person skilled in the pertinent art.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a physician, doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density are typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the term "biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. A system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic pulse is described herein, it is understood that the expressed voltage amplitude is an absolute value of the approximate peak amplitude of each of the positive-voltage phase or the negative-voltage phase. Each phase of the biphasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

In an electrophysiology system, electrical activity within the heart is detected using a catheter with two or more electrodes for bipolar mapping. Electrical activity within the heart is detected using a first electrode configured for sensing both local activation energy (near-field signals) at the point of contact with heart tissue and far field activation energy (far-field signals) received by the electrode through the blood. In close proximity to the first electrode, the second electrode is configured to receive approximately the same far-field signals but not the local activation energy (near-fields signals). A suitable signal processing unit processes the signals received by both the first and second electrodes and by subtracting the far-field signals detected by the second electrode from the near- and far-field signals detected by the first electrode, near-field signals can be more accurately determined.

The catheter typically also comprises a location sensor, e.g., an electromagnetic position sensor. Suitable electromagnetic sensors are described in U.S. Patent Nos. 5,443,489, 5,480,422, 5,546,951, 5,568,809 and 5,391,199, the disclosures of which are incorporated herein by reference. In some embodiments, to use the electromagnetic sensor, the patient is placed in a magnetic field generated, for example, by situating under the patient a pad containing coils for generating magnetic field(s). A reference electromagnetic sensor is fixed relative to the patient, e.g., taped to the patient's back, and the catheter with the electromagnetic location sensor is advanced into the patient's heart. Each sensor preferably comprises three small coils which in the magnetic field(s) generate electrical signals indicative of their position in the magnetic field(s). Signals generated by both the fixed reference sensor and the sensor in the catheter in the heart are processed to ascertain a precise location of the sensor in the catheter relative to the reference sensor. Using this technology, a physician can visually map a heart chamber. This mapping is done by advancing the catheter into a heart chamber until a distal tip makes contact with the heart wall. This position is recorded and saved. The distal tip is then moved to another position in contact with the heart wall and again the position is saved. By combining the electromagnetic sensor and sensing electrodes, a physician can simultaneously map the contours or shape of the heart chamber and the electrical activity of the heart and generate 3-D electroanatomical maps for display on a monitor. Errant electrical activities of the patient's heart may therefore be viewed and diagnosed by the physician.

The 3-D electroanatomical map may also be based on an estimated anatomical map. Mapping algorithms that are based on such measurements, such as fast anatomical mapping (FAM), are known in the art. The FAM method may provide a physician with additional mapping capabilities, such as electro-physiological (EP) mapping that may be used for cardiac ablation. During the FAM procedure, a physician navigates the distal end of the catheter to desired locations in the heart to collect anatomical signals therefrom. In principle, the FAM may provide the physician with a surface representing an estimated anatomical mapping of the tissue in question. Point positions on the surface of a heart chamber are drawn using acquired electroanatomical data. This surface will be used by the physician during EP mapping and ablation procedures.

Catheters may also be configured to provide hybrid magnetic-based and impedance-based position sensing in benefitting from both the higher accuracy of magnetic position sensing and the lower cost of impedance-based sensing. In impedance-based active current location (ACL) sensing, impedance is measured between electrodes on the catheter and external skin patch electrodes placed on the patient's body. An electrical signal is applied to the electrodes on the catheter ("active" electrodes) and the resulting voltages and/or currents are measured at the external skin patch electrodes ("return" electrode). In hybrid position sensing, externally-applied magnetic fields are measured by the magnetic field sensor, and accurate position coordinates of the catheter are derived. Currents or voltages from the external skin patch electrodes are also applied, and impedances between the external skin patch electrodes and the catheter electrodes are measured. The dual position measurements are repeated at multiple locations within the body cavity in order to generate a calibration map, correlating the impedance measurements with position coordinates ascertained by the magnetic field sensor. Additional catheters with diagnostic or therapeutic functions may be introduced into the heart, and these additional catheters need not include magnetic field sensors, as impedance measurements taken from electrodes of these additional catheters are correlated with the calibration map in order to determine accurate position coordinates of these additional catheters. Notably, typical values of frequency and amplitude of the active-current-location ("ACL") signals are on the order of 100 kilohertz (kHz) and 1 millivolts (mV), respectively, whereas the respective values of frequency and amplitude of the ECG signals are on the order of 1 hertz (Hz) and 1 microvolt (µV).

An ablation system typically comprises a catheter with at least two electrodes coupled to an energy source. One of the electrodes is configured as an anode and the other as a cathode. Electrodes may be energized with DC voltages and conduct currents are various frequencies, amplitudes, pulse widths and polarities. When the energy source supplies an energizing potential to an electrode, an electrical current is conducted between the first and second electrodes through patient tissue. Polarity of the electrodes may be reversed by reversing the polarity of the output of the energy source. The electric current supplied by the energy source may comprise pulses or pulse sequences. Each pulse may be biphasic including a first component having a polarity and a second component having an opposite polarity. Pulses may include blended unipolar/bipolar pulses.

Irreversible electroporation (IRE), also called Pulsed Field Ablation (PFA), may be used as an invasive therapeutic modality to kill tissue cells by subjecting them to high-voltage pulses. Specifically, IRE pulses have a potential use to kill myocardium tissue cells in order to treat cardiac arrhythmia. Cellular destruction occurs when the transmembrane potential exceeds a threshold, leading to cell death and thus the development of a tissue lesion. Therefore, of particular interest is the use of high-voltage bipolar electric pulses, e.g., using a selected pair of electrodes in contact with tissue to generate high electric fields, e.g., above a certain threshold to kill tissue cells between the electrodes.

Embodiments of the present invention that are described hereinafter use a multi-electrode catheter configured for both mapping and ablation, including RF, PFA and IRE ablation of IRE pulses with typical magnitudes of 1kV - 3kV, operable in both unipolar and bipolar modes. The catheter may include a balloon, basket, or lasso, with coated electrodes adapted to lower impedance and increase electrical conductivity for improved delivery of current for ablation. Moreover, the catheter enables selective electrical connection or "shorting" of multiple electrodes together, for example, electrodes on selected spines, based on detection of tissue contact at each electrode, for ablating solely at the electrodes where needed and minimizing the application of excessive current to patient. Selective "shorting" of these electrodes effectively forms one composite catheter electrode for more efficient ablation.

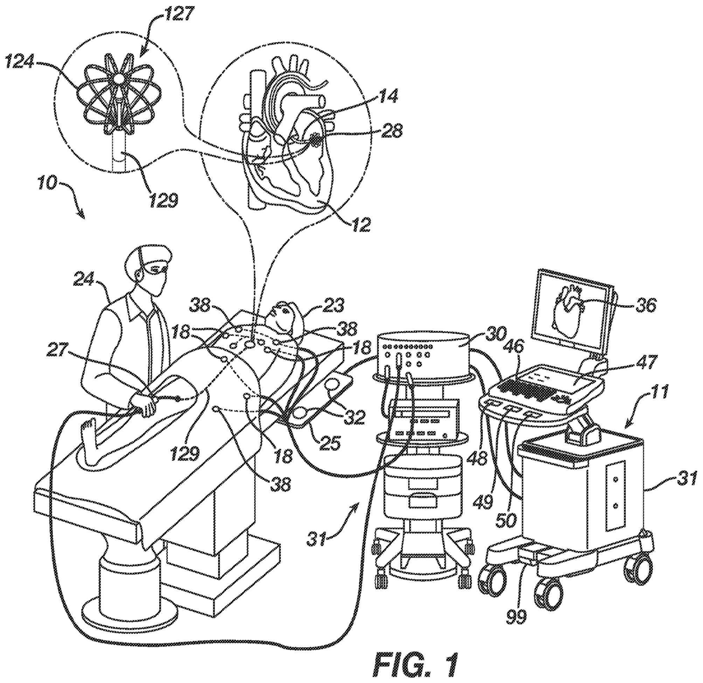

**FIG. 1** is a schematic, pictorial illustration of a catheter-based irreversible electroporation (IRE) system 10 using a basket catheter 27 wherein a shaft 129 of the catheter 27 is inserted by a physician 24 through the vascular system of a patient 23. The catheter is configured so that a physician can navigate an end effector or expandable basket electrode assembly 127 of the catheter 27 to a target location inside the heart 12 of the patient 23.

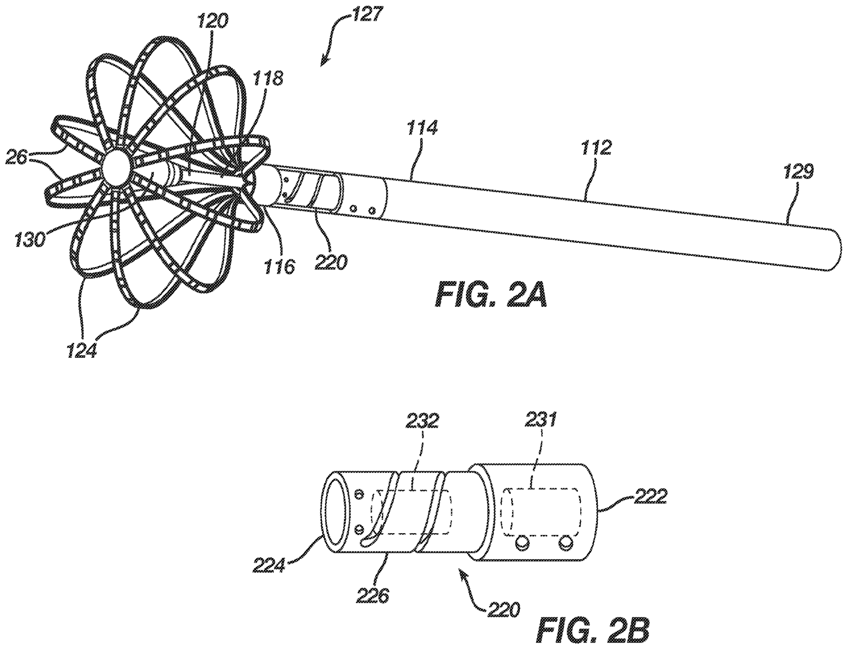

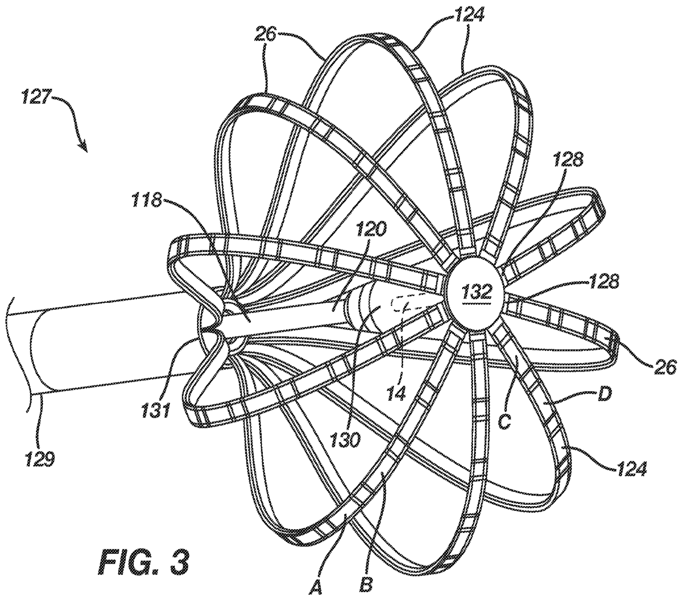

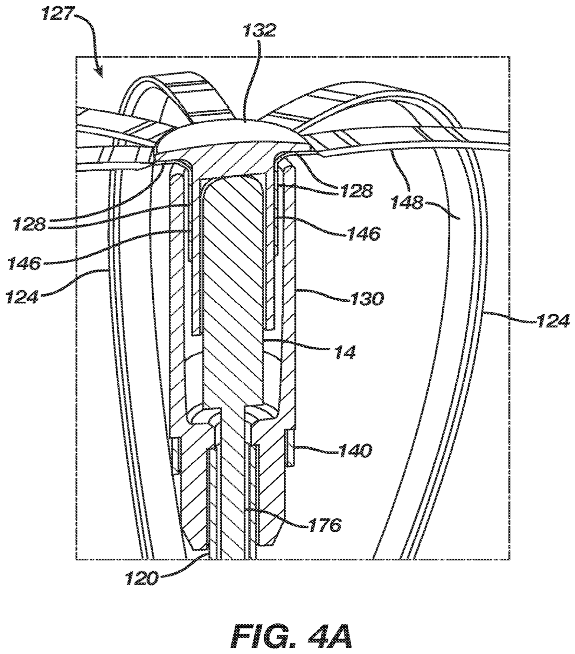

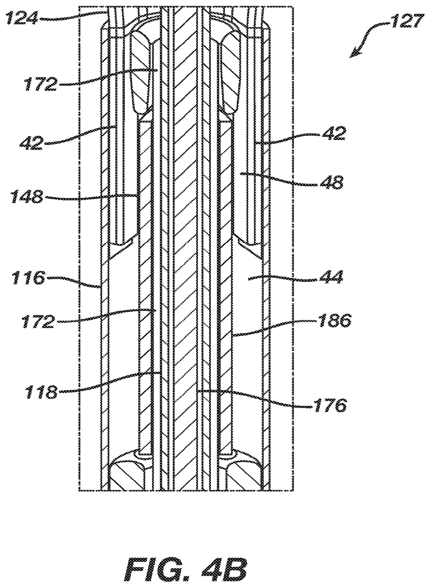

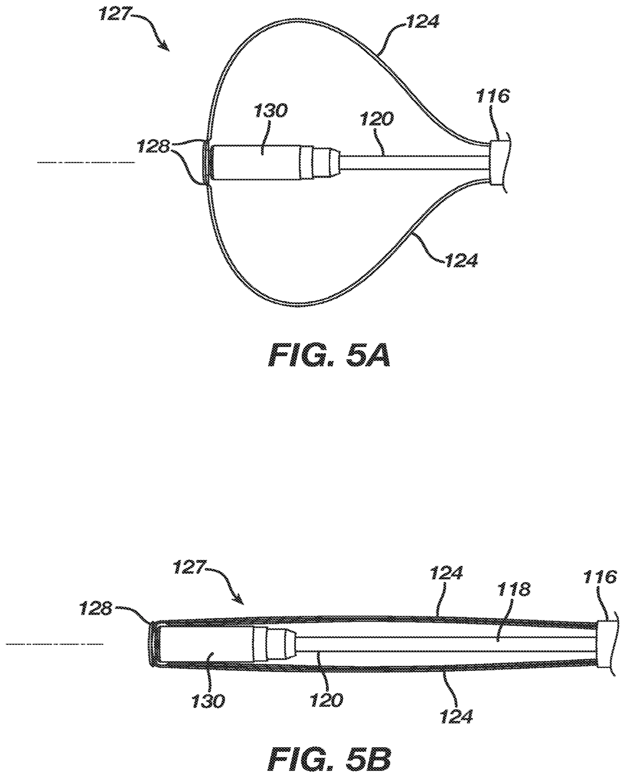

In the embodiment of **FIG. 2A** and **FIG. 3****,** the shaft 129 of the basket catheter 27 includes a deflectable section 112 and the basket catheter 27 includes an elongated pusher 118 that extends through the shaft 129 and the deflectable section 112 and is longitudinally movable relative thereto. The pusher 118 has a distal section 120 that acts on a distal end of the basket assembly 127 such that the physician 24 can retract the pusher 118 relative to the shaft 129, for example, using a manipulator or handle (not shown), to expand the basket assembly 127, as shown in **FIG. 5A** or extend the pusher 118 to collapse the basket assembly, as shown in **FIG. 5B****.** The basket assembly 127 includes a plurality of flexible polymer circuit strips 124. Each flexible polymer strip 124 includes multiple electrodes 26 disposed thereon. With reference to **FIG. 4A** and **FIG. 4B****,** the basket catheter 27 includes a nose connector 130 that is connected to the distal portion 120 of the pusher 118. The flexible polymer circuit strips 124 are connected to the nose connector 130 via hinges 128 formed at distal ends 146 of the flexible polymer circuit strips 124. The distal ends 146 are devoid of electrodes 26 as they are sandwiched between a nose cap 132 and the nose connector 130. The basket catheter 27 also includes respective elongated resilient support elements 148 disposed along a given length of respective ones of the flexible polymer circuit strips 124 providing a shape of the basket assembly 127 in the expanded form of the basket assembly 127.

As shown in **FIG. 5A** and **FIG. 5B****,** the flexible polymer circuit strips 124 are configured to bow radially outward when the pusher 118 is retracted by the physician in expanding the basket assembly 127 from a collapsed form and to extend generally parallel with the pusher 118 when the pusher 118 is advanced by the physician in collapsing the basket assembly from an expanded form. The collapsed form of the expandable assembly 127 represents the non-stressed form of the flexible polymer circuit strips 24 which are provided with their shape by the elongated resilient support elements 148.

In some embodiments, the distal portion 120 of the pusher 118 houses a multi-axis position sensor 14 (**FIG. 4A**) which may comprise a dual-axis or triple-axis position sensor, for example, a magnetic position sensor comprising multiple orthogonal coils. Wiring 176 to transmit position signals is connected the multi-axis position sensor 14 via the hollow of the pusher 118. The nose cap 132 covers the nose connector 130 mounted on the distal portion 120 of the pusher 118, retaining the multi-axis position sensor 14 in the nose connector 130, while also securing the distal ends 146 of the strips 124 to the nose connector 130 and hence the distal portion 120 of the pusher 118. An irrigation sleeve 172 (**FIG. 4B**) is disposed in the shaft 129 with a distal end being generally coterminous with a distal end 131 of the shaft 129 so that irrigation fluid can pass through the shaft and exit at the distal end of the shaft. A single-axis position sensor 186 is disposed circumferentially around the irrigation sleeve 172 at a location immediately proximal of the distal end 131. The signal axis sensor 186 allows for the navigation system to locate the distal end of the catheter. Similar basket catheters are described in U.S. Publication Nos. 2021/0187241 and 2023/0346459, the entire disclosures of which are incorporated herein by reference.

After the basket assembly 127 has reached the target location as shown in **FIG 1****,** the physician retracts the pusher 118 to expand the basket assembly 127. The physician 24 then manipulates the shaft 129, including the deflectable section 112 to deflect the basket assembly unidirectionally or bidirectionally, such that one or more electrodes 26 disposed on the basket assembly 127 come into contact with tissue of the heart 12 to sense electrical activity, including intracardiac electrogram (IEGM) signals, map a chamber of the heart 12 and/or apply voltage, including high-voltage PFA pulses for irreversible electroporation (IRE), via the electrodes 26.

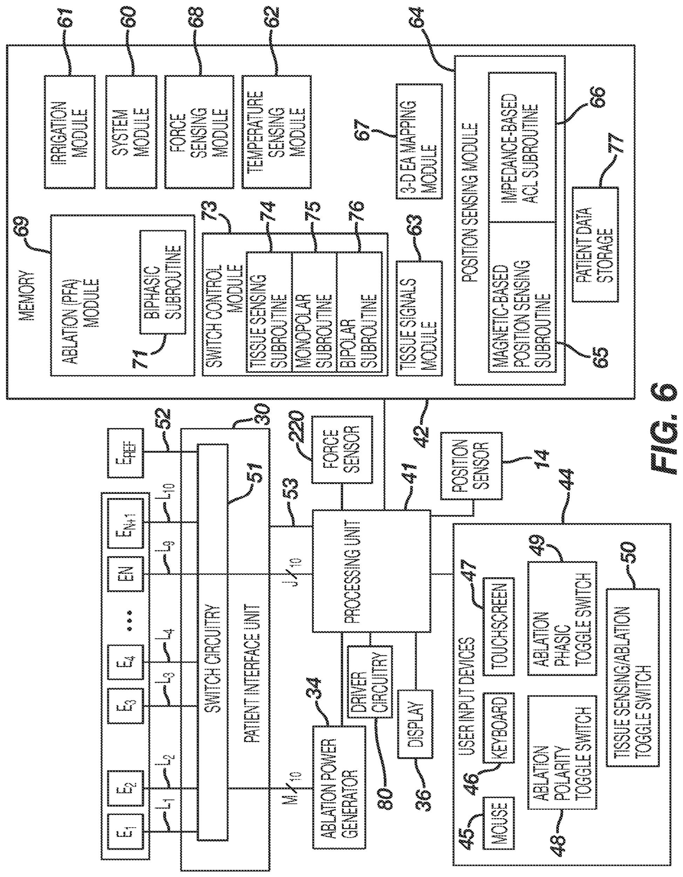

With reference to **FIG. 6****,** the system 10 includes an operating console 31 which may include a patient interface unit (PIU) 30, an ablation power generator 34 and a display 36 to display 3-D maps and electrograms. The ablation power generator 34 is adapted to conduct ablative energy to ablation electrodes. Energy produced by ablation energy generator may include, but is not limited to, radiofrequency (RF) energy or pulsed-field ablation (PFA) energy, including unipolar or bipolar high-voltage DC pulses as may be used to effect irreversible electroporation (IRE), or combinations thereof. The PIU 30 is an interface configured to establish electrical communication between the catheter, the electrophysiological equipment, a power supply and a system controller for controlling operation of system 10. In some embodiments, the PIU 30 additionally includes processing capability for implementing real-time computations of location of the catheters and for performing ECG calculations.

The operations, functions and acts of the system 10 are managed by a system controller 11 that includes a processing unit 41 communicating with a memory 42 wherein is stored software for operation of the system. In some embodiment, at least some of the operations, functions or acts of the system controller 11 are performed using custom-designed hardware and software, such as an application specific integrated circuit (ASIC) or a field programmable gate array (FPGA). In some embodiments, the system controller 11 is managed by the physician using the user interface devices, which enable the physicians to set parameters of the system. Results of the procedure are provided to the physician on display 36. The software in memory 42 may be downloaded to the system controller 11 in electronic form, over a network, for example. Alternatively or additionally, the software may be provided on non-transitory tangible media, such as optical, magnetic or electronic storage media.

As shown in **FIG. 6****,** in some embodiments, the memory 42 includes multiple modules used by the processing unit 41, such as, for example, a system module 60 for controlling the modules in the memory 42 and the processing unit 41 in executing the modules, an irrigation module 61 for controlling fluid supply and flow through the catheter, a temperature sensing module 62 for sensing temperature at the tissue target location, a sensing tissue signals module 63 for sensing heart electrical signals, such as IEGM signals for generating electrograms, and a position sensing module 64 to track position (location and orientation) of the distal end 22 of the catheter, that may include a magnetic-based position sensing subroutine 65 responsive to external magnetic field(s) or it may also include an impedance-based ACL subroutine 66 for position sensing based on a hybrid of electromagnetic positioning sensing and impedance-based active current location (ACL) sensing. The memory 42 may also include a 3D EA mapping module 67 for generating 3-D electroanatomical maps of the heart that indicate cardiac electrical activities and location of such activities, a force sensing module 68 for sensing contact between the distal end 22 of the catheter and heart tissue 12, and an ablation (PFA) module 69 for controlling and applying ablation, including PFA, to heart tissue, that includes a biphasic subroutine 71. The memory 42 may further include a switch control module 73 with a tissue sensing subroutine 74 for controlling switch connections in the electrode electrical circuits for sensing tissue electrical signals, and a unipolar subroutine 75 and a bipolar subroutine 76 for controlling switch connections in the electrode electrical circuits as "active" or "return" electrodes during ablation. In some embodiments, the memory 42 also includes a patient data storage 77 for storing data received and/or generated by the system.

In some embodiments, the system controller 11 includes an industry-standard personal computer 43 including a general-purpose computer processing unit that is responsive to user input devices 44, including, for example, a mouse 45, a keyboard 46, a touchscreen 47 and/or foot pedal 99. Additional user input devices may include an ablation polarity toggle control 48 to switch between unipolar ablation mode and bipolar ablation mode, and a toggle control 50 to switch between tissue electrical sensing mode and ablation mode.

In some embodiments, the PIU 30 of the system controller 11 includes a circuit connection device, including, for example, a switch circuitry 51 comprising switches and/or relays, responsive to the controls 48, 50 of the user input devices 44 in actuating electrical connection/disconnection of selected catheter electrodes 26 to the ablation power generator 34 or to the processing unit 41 in rendering the electrodes 26 as "active" or "return" in the electrical circuit used for the user-selected modes of operation between tissue electrical activity sensing, unipolar ablation and bipolar ablation. As shown in the embodiment of FIG. 6, each electrode E1 - EN+1 is coupled to the switch circuitry 51 via a respective lead L1 - LN+1, and further to the ablation power generator 34 via a respective lead M (collectively designated as M/N+1), and to the processing unit 41 via a respective lead J (collectively designated as J/N+1). A reference electrode Eref is also connected to the switch circuitry 51 by a lead 52 and further to the processing unit 41 by a lead 53. As shown in FIG. 4A, a center ring electrode 140 is mounted on an outer surface of a proximal portion of the nose connector 130. Surrounded by the strips 124, the center ring electrode 140 is generally blocked from tissue contact and thus can function as a reference electrode as needed or appropriate.

Selected electrical connections/disconnection of the electrodes 26 to the ablation power generator 34 and/or the processing unit 41 via the switch circuity 51 is conditioned on detection of contact between the electrodes 26 and heart tissue 12, and/or the degree of such contact exceeding a predefined threshold force, by the contact force sensor assembly 220 which is coupled to the processing unit 41 and configured to detect contact between tissue and the basket assembly, including contact between one or more respective electrodes and heart tissue.

The basket catheter 27 is configured for multiple functions, including sensing tissue electrical signals and ablating tissue. As shown in **FIG. 1****,** the physician brings the basket assembly 127 of the catheter 27 into contact with the heart wall to sense a target site in heart 12. For ablation, physician 24 similarly brings the basket assembly 27 into contact with the heart wall at the target site. For tracking position of the basket assembly 27, the catheter includes the multi-axis position sensor 14 housed in the nose connector 130 at the distal end of the pusher 118 (**FIG. 3** and **FIG. 4A**). In some embodiments, the position sensor 14 is a magnetic-based position sensor with three magnetic coils for sensing three-dimensional (3D) position and orientation. The magnetic-based position may be operated together with a location pad 25 that includes a plurality of magnetic coils 32 configured to generate magnetic fields in a predefined working volume. Real time position of the basket assembly 127 may be tracked based on magnetic fields generated with location pad 25 and sensed by magnetic based position sensor 14. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The system 10 includes one or more electrode patches 38 positioned for skin contact on patient 23 to establish location reference for location pad 25 as well as impedance-based position tracking of electrodes 26. For impedance-based position tracking, the system control 11 actuates a driver circuitry 80 to send an electrical current to each electrodes 26 which is sensed at each electrode skin patches 38 and used to measure the impedance between the electrodes 26 and the electrode skin patches 38. Based on the measured impedances, the processing unit 41 can access the impedance-based ACL subroutine 66 of the position module 64 to determine the position of the basket assembly 127 relative to the electrode skin patches 38. Details of the impedance-based location tracking technology are described in US Patent Nos. 7,536,218; 7,756,576; 7,848,787; 7,869,865; and 8,456,182.

As shown in FIG. 2A, a contact force sensor assembly 220 is disposed in the shaft 129 proximal of the basket assembly 127. In the illustrated embodiment of FIG. 2B, the force contact sensor assembly 220 includes a proximal end 222 and a distal end 224 with the proximal end 222 housing a magnetic field transmitter coil 232 and the distal end 224 housing a magnetic field receiver 232. As understood in the art, the magnetic field transmitter coil 232 can be configured to generate a magnetic field while the magnetic field receiver coils 231 can be configured to detect the presence and magnitude of the magnetic field. The contact force sensor assembly 220 can further include an elastically-deformable deflection portion 226 disposed between the proximal end 222 and the distal end 224 and configured to deflect when a force is applied to the contact force sensor assembly 220. The deflection portion 226 can be configured to permit a spatial relationship between proximal end 222 and the distal end 224 to change when a force is applied. In the illustrated embodiment, the deflection portion 226 includes a helical spring 226 with a distal edge 260 of the tube 240 includes openings 262 (only some labeled for the sake of simplicity) disposed around the circumference of the distal edge 260. The inner proximal surface 258 of the distal coupler 226 includes protrusions 264 (only some labeled for the sake of simplicity), disposed circumferentially around the inner proximal surface 258, and configured for engaging the openings 262 (or slots disposed around the perimeter of the generally tubular member 38) to prevent rotation of the distal coupler 226 with respect to the tube 240. The openings 262 and the protrusions 264 may be any suitable shape. In some embodiments, the openings 262 include U-shaped openings as shown in Fig. 2B.

A change in the spatial relationship when the deflection portion 226 is deformed due to the application of a contact force on the basket assembly 127 results in the magnetic field receiver coils 231 detecting a change in the magnetic field (due to movement of the sensor 232). Because the spring constant K of the deflection portion 226 can be predetermined and the distance between the magnetic field transmitter coil 232 and the magnetic field receiver coils 231 can be detected, the force applied to the basket assembly 127 can be determined. And where the magnetic field receiver coils 231 includes at least three magnetic sensing coils, an angular direction and a magnitude of a displacement between the magnetic field transmitter coil 232 and the at least three magnetic sensing coils 231 5can be determined via triangulation. Suitable contact force sensors are described in U.S. Publication No. 2023/0346459, the entire disclosure of which is incorporated herein by reference. Also predetermined is the spatial relationship of each of the electrodes 26 on the basket assembly 127 relative to the contact force sensor assembly 220 as the configuration of the basket assembly 127 and the arrangement of the flexible polymer circuit strips 124 and the electrodes 26 thereon are known. Thus, identity of those electrodes 26 on the basket assembly 127 in tissue contact can be inferred when the contact force sensor assembly 220 detects a directional and distance displacement of the proximal end 222 relative to the distal end 224.

The inference of contact between any particular electrodes 26 and tissue by the contact force sensor 220 can be confirmed by impedance measurements. As mentioned, impedance can be measured between the electrodes 26 and the electrode skin patches 38, as shown in FIG. 1. In addition to the system 10 determining catheter position by using impedance measurements between the electrodes 26 and the patches 38, the system controller 11 can also infer tissue contact via driver circuitry 80 to drive a current between each electrode 26 and the corresponding body surface electrode 38 to measure the impedance between them in determining whether each electrode is in contact with tissue. It is understood that impedance increases when an electrode in contact with blood moves into contact with tissue so the system 10 can use impedance measurements in combination with detection of displacement by the contact force (and direction of force) sensor assembly 220 to at least infer, if not determine, which electrodes 26 on the basket assembly 127 are in contact with tissue. In other embodiments, body surface electrodes 38 may take other forms, such as subcutaneous probes or handheld device operated by medical professional.

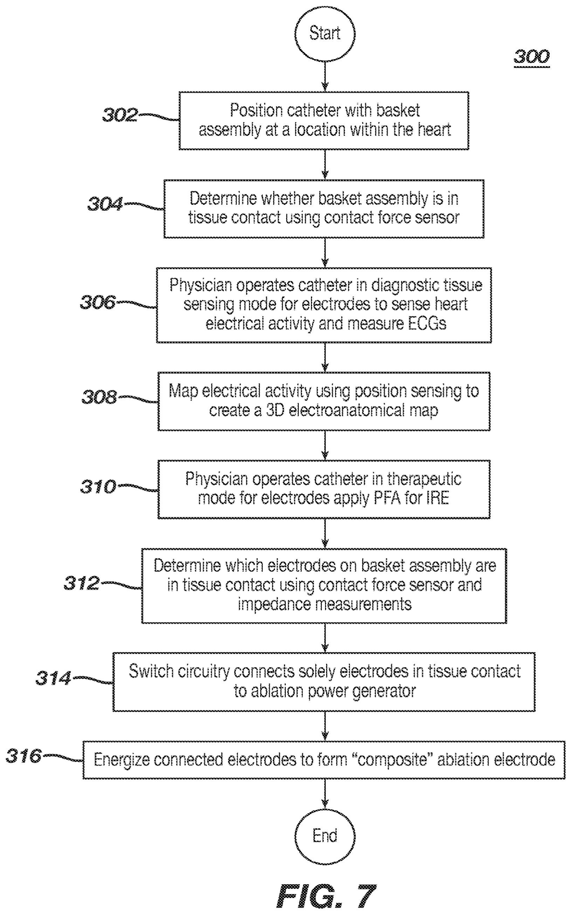

FIG. 7 is a flowchart illustrating a method 300 of diagnosing and treating heart arrhythmia, in accordance with an embodiment of the present invention. At Block 302, method 300 includes positioning the catheter 27 with the basket assembly 127 at a location within the heart. The physician 24 can deflect the catheter 27 using the deflection section 112 in maneuvering the basket assembly 127 into position at the location in the heart.

At Block 204, the method includes determining whether the basket assembly 127 is in tissue contact with the heart via the contact force sensor 220 (Block 304). In some embodiments, with reference to FIG. 6, the processing unit 41 accesses the force sensing module 68 in actuating the contact force sensor 220. As the deformable portion 226 of the sensor 220 bends or otherwise deforms upon the external force exerted on the basket assembly 127 by the heart tissue, the sensor 220 generates signals indicating contact between the basket assembly 127 and the heart tissue.

At Block 306, the physician actuates operation of the catheter 27 in a diagnostic tissue sensing mode via the switch 50 for electrodes 26 to sense heart electrical activity. In some embodiments, the processing unit 41 accesses the tissue sensing subroutine 74 of the switch control module 73 and actuates the switch circuitry 51 for the appropriate switch connections between the electrodes E1-EN+1 and the processing unit 41 to receive the sensed heart electrical activity for measuring ECGs.

At Block 308, the processing unit 41 accesses the 3D EA mapping module 67 to create a 3D electroanatomical map for display on the display 36. The processing unit 41 having access to the memory or storage with appropriate operating software loaded therein, and user interface capability may include modeling the endocardial anatomy in three-dimensions (3D) and rendering the model or anatomical map 20 for display on the display device 36. Other functions may include displaying activation sequences (or other data) compiled from recorded electrograms in representative visual indicia or imagery superimposed on the rendered anatomical map, displaying real-time location and orientation of the catheter within the heart chamber, and displaying sites of interest such as places where ablation energy has been applied. One commercial product embodying elements of the system 10 is available as the CARTO ^{™} 3 System, available from Biosense Webster, Inc., 31 Technology Drive Suite 200, Irvine, CA 92618 USA.

In some embodiments, the processing unit 41 uses the sensed heart electrical activity and 3D mapping of the heart via the multi-axis position sensor 14 housed in the nose connector 130 inside of the basket assembly 127 and the magnetic-based position sensing subroutine 65 of the position sensing module 64. Impedance-based ACL subroutine 66 may also be employed for determining the position of the basket assembly 127. The 3D electroanatomical map should reveal the region(s) of irregular electrical signals in the heart, for example, arrhythmias.

At Block 310, the physician actuates operation of the catheter in a therapeutic mode via the switch control 50 for electrodes E1-EN+1 to receive ablation energy and ablate heart issue in contact with the electrodes. The control 50 is a toggle which switches between tissue sensing (e.g., mapping) mode and ablation mode and may be for example, a hand control switch or a foot pedal. In some embodiments, the processing unit 41 accesses the ablation module 69 and actuates the switch circuitry 51 for the appropriate switch connections between the electrodes and the ablation power generator 34 for delivery of ablation energy. In this regard, ablation energy is delivered solely to those electrodes in contact with heart tissue which is determined by the contact force sensor 220 assembly. And, where a displacement between the proximal and distal ends 222, 224 of the contact force sensor assembly 220 is defined and measured in terms of a distance and an angular direction by the magnetic field receiver coils 231 in response to the magnetic field transmitter 231, the processing unit 41 employing the force sensing module 68 which has access to the configuration of the basket assembly 127 and the location of each electrode E1-EN+1 thereon within that configuration can infer which electrode(s) are in contact with the heart tissue. In some embodiments, that inference can be confirmed by impedance measurements with the understanding that impedance increases when electrodes are in contact with heart tissue and decreases when electrodes are not in contact with heart tissue. For example, the impedance measurements can be obtained by the processing unit 41 in accessing the impedance-based ACL subroutine 66 of the position sensing module 64 to actuate the switch circuitry 51 for electrical connection between each electrode 26 and the processing unit 41 which then sends an electrical current to each electrode 26 that is received by the processing unit 41 in measuring the impedance of each electrode. By combining the displacement measurement of the contact force sensor assembly 220 and the tissue contact via impedance measurement of each electrode, the processing unit 41 can identify which electrodes are in contact (as well as the approximate contact force and direction of force being applied to the electrode(s)) with the heart tissue and actuate the switch circuitry 51 accordingly to disconnect electrical connection from the ablation power generator 34 to those electrodes not in contact with heart tissue and connect solely those electrodes in contact with heart tissue to the ablation power generator 34 in forming one or more composite electrodes for delivering PFA to selected regions in the heart. For example, with reference to FIG. 3, the processing unit 41 may identify electrodes A, B, C and D to be in contact with heart tissue and actuate the switch circuitry 51 to connect electrodes A, B in forming a first composite electrode and electrodes C, D in forming a second composite electrode, wherein the first composite electrode is an active electrode and the second composite electrode is a return electrode for bipolar ablation, or wherein the first and second composite electrodes are both active electrodes and a third electrode, e.g., the center ring electrode 140 or a body surface patch 38, is a return electrode for unipolar ablation. Selective electrical connection of electrodes enable smaller electrodes to be combined to deliver ablation energy of higher voltages for PFA and IRE without damage to the basket assembly or exposing the patient to excess ablation energy.

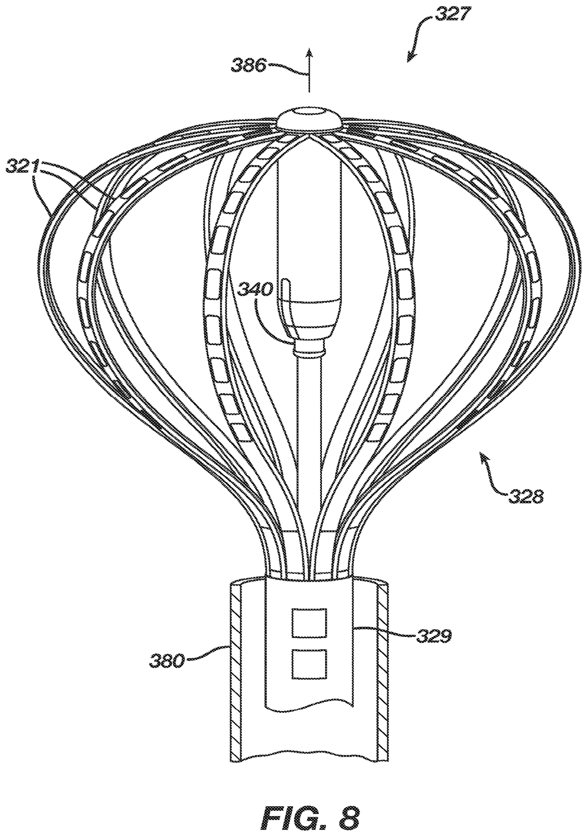

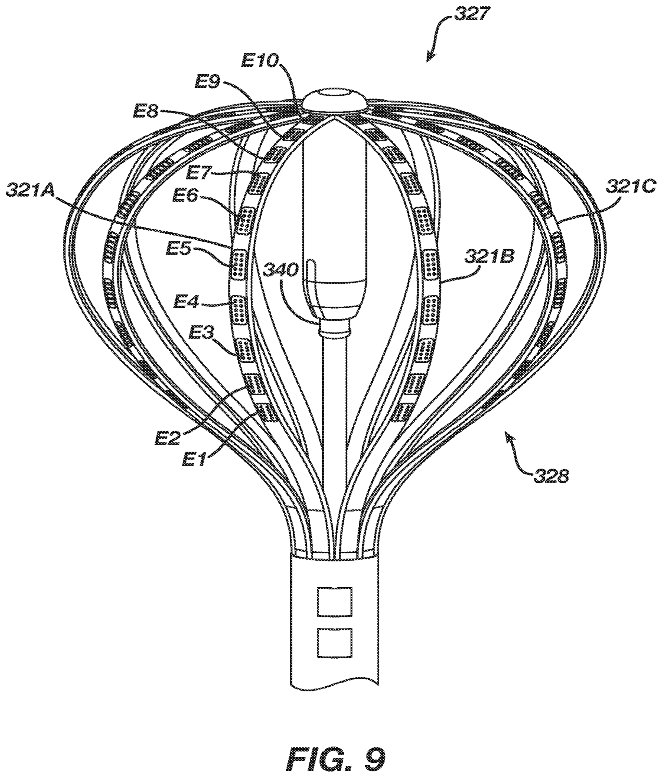

FIG. 8 and FIG. 9 depict a catheter 327 with a basket assembly 328 in an expanded form when unconstrained, such as by being advanced out of a distal end 381 of a sheath 380. The basket assembly 328 includes a plurality of flexible spines 321 extending past the end of a shaft 329. In the illustrated embodiment, the spines 321 converge at both a distal end and a proximal end of the basket assembly 328. During a medical procedure, a physician can deploy the basket assembly 328 by advancing the shaft 329 distally relative to the sheath 380 causing the basket assembly 328 to exit the sheath 380 and transition from a collapsed form to the expanded form. In the expanded form, the spines 321 of the basket assembly 328 bow radially outwardly along a longitudinal axis 386 and in the collapsed form the spines 321 are constrained generally along the longitudinal axis 386 of the shaft 329 of the catheter 327.

With reference to FIG. 8 and FIG. 9, it is noted that each of electrodes E1-E10 can be coated with an impedance reduction coating as described in detail in U.S. Patent Application Serial No. 18/159,288, which is incorporated by reference as if set forth in full herein with a copy attached to the Appendix. Each flexible strip 321 may be constructed of a flexible polymer circuit strip 321 that includes a polyimide layer. Hinges 328 of the flexible polymer circuit strips may be strengthened with any suitable material, for example, but not limited to, a length of yarn, which is flexible and provides tensile support to the strips. In some embodiments, a length of yarn runs the whole length of each strip including the hinges. The yarn may include any suitable yarn. For example, the yarn may include one or more of the following: an ultra-high-molecular-weight polyethylene yarn; or a yarn spun from a liquid-crystal polymer. Each flexible polymer circuit strip, its length of yarn, and elongated resilient support element may be secured together with a suitable adhesive, for example, epoxy, and then covered with a thermoplastic polymer resin shrink wrap (PET) or any other suitable covering. Windows (or apertures) may be created in the PET covering with a laser, mechanical removal, or any other suitable method in order to expose the electrodes. Alternatively, prior to shrinking, the PET covering may already have windows present. The flexible polymer circuit strips may further include a conductive polymer coating, such as poly(3,4-ethylenedioxythiophen) (PEDOT) or poly(3, 4 ethylenedioxythiophene) polystyrene sulfonate (PEDOT:PSS), over each electrode to help protect the electrode, reduce input impedance, and enhance the signal-to-noise ratio. The conductive polymer coating may be applied to each electrode by dipping the electrode in a solution comprising the conductive polymer coating and then passing an electrical current through the electrode. As the current passes through each electrode, the conductive polymer coating adheres to the surface of the electrode. To help reduce the likelihood that the conductive polymer coating is damaged by rubbing on the sheath or contacting other objects, the disclosed technology can include forming apertures in the PET covering with a laser, mechanical removal, or any other suitable method in order to expose only a portion of each electrode. In other words, rather than removing the PET covering to expose the entire surface of the electrode, the disclosed technology can include removing smaller portions of the PET covering to form small apertures through the PET covering to expose only portions of the electrode's surface. By including small apertures through the PET, the PET can provide protection to the conductive polymer coating which is positioned in each aperture by preventing the conductive polymer coating from contacting the sheath or other objects. The apertures can be sized, shaped, and positioned to help reduce the likelihood that the conductive polymer coating will contact the sheath or other objects while also ensuring the electrode is capable of detecting electrical signals of the heart. Reduction in damage to the conductive polymer coating may result in more accurate signals from the electrodes and/or less risk of a health threat due to shedding of coating into the patient's heart and/or vasculature. Details of the rope or yarn, the PET shrink wrap, windows in the PET shrink wrap and conductive polymer coating are shown and described in applicant's published patent application US20210187254A1 (Attorney Docket No. BIO6243), which is incorporated by reference with a copy in the Appendix.

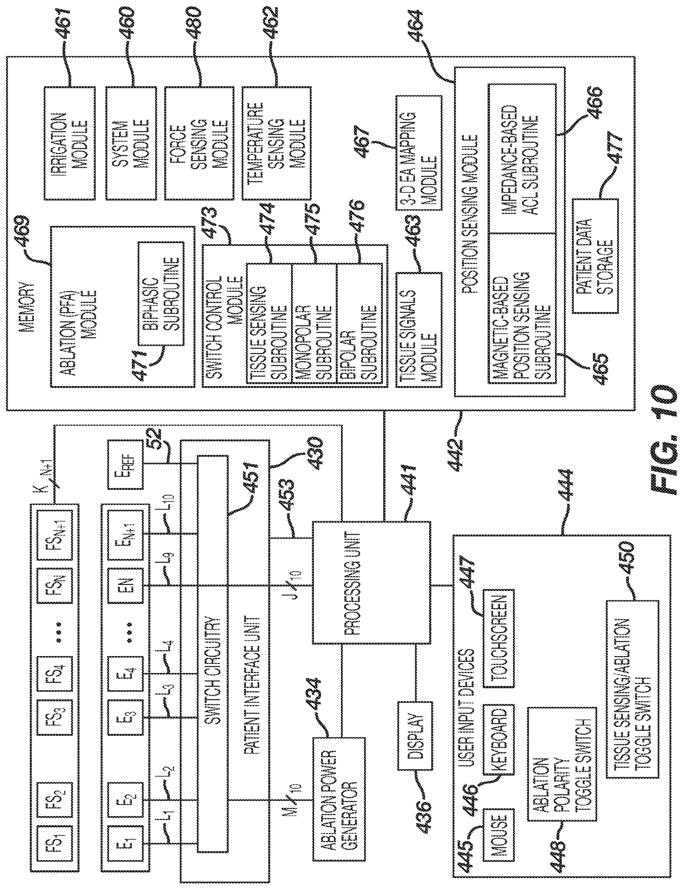

With reference to FIG. 10, a system 410 includes a switch circuitry 451 which may be provided in a PIU that establishes electrical communication between a catheter with the basket assembly 328, an ablation power generator 434 and the processing unit 441. The switch circuitry 451 is configured to be responsive to user input devices 444 in actuating electrical connection/disconnection of selected electrodes E₁ - E_{N+1} (e.g., electrodes E1 - E10 of FIG. 8 and FIG. 9) of the basket assembly 328, the ablation power generator 434 and/or the processing unit 441 in rendering these electrodes as "active" or "return" in the electrical circuit used for the user-selected modes of operation between tissue electrical activity sensing (e.g., mapping), unipolar ablation and bipolar ablation. Such selected electrical connections/disconnection may be further conditioned on detection of contact between the electrodes E1-E10 of the basket assembly and heart tissue, and/or the degree of such contact exceeding a predefined threshold force, by a force sensor assembly 320 in the manner described hereinabove. Alternatively, the basket assembly 328 may include_a plurality of force sensors FS1 - FS10, each of which is configured to detect contact between a respective electrode Ei and heart tissue 412, where the force sensors FS1-FS10 can be disposed at, for example, either the distal ends or the proximal ends of the spines. Thus, the switching circuitry 451 can create from selected electrodes on the catheter one or more effective composite electrodes by short-circuiting one to the other only those electrodes detected to be in contact with tissue and disconnecting those electrodes not detected to be in contact with tissue or not to be in sufficient contact with tissue in terms of a predetermined threshold contact force, in order to avoid applying excessive current to the patient and applying current only where needed.

In the embodiment of **FIG. 10****,** a switch circuitry 451 includes switches SW1 - SW10, providing for each electrode a respective three-way switch (or double throw switch) that is configured to connect the respective electrode to one of at least three settings at any given time. The three settings of each switch SWi are:
(i) setting S1 to connect to an ablation power generator 434 as an "active" electrode for ablation, including unipolar and bipolar ablation,
(ii) setting S2 to connect to the processing unit 41 as a "return" electrode for sensing heart electrical signals and for bipolar ablation, and
(iii) setting S3 to connect to a float F as an inactive electrode not in contact or in insufficient contact with tissue in either unipolar or dipolar ablation.

In some embodiments, the basket assembly 328 also includes an electrode ERef, for example, a ring electrode disposed in the center of the basket assembly, e.g., on a center longitudinal tube 321 extending between the proximal and distal end of the basket assembly. The electrode ERef has a respective two-way switch SWW which provides (i) setting S5 to connect to the processing unit as a "return" electrode or TRUREF^{™} electrode when the system is operating in the unipolar ablation mode, and (ii) setting S6 to connect to a float as an inactive electrode.

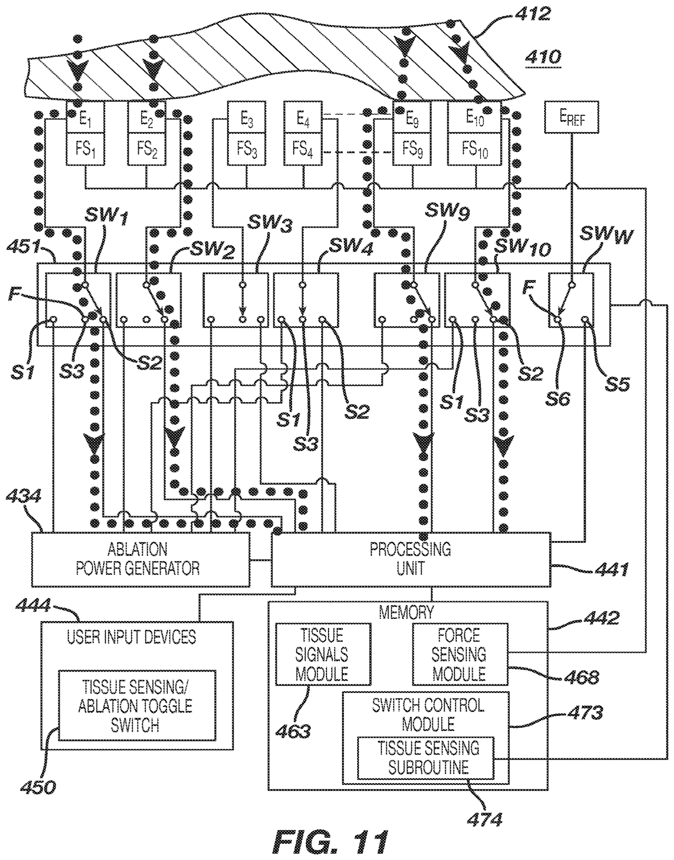

With reference to FIG. 10, FIG. 11, FIG. 12 and FIG. 13, only one spine of the basket assembly and only electrodes E1, E2, E3, E4, E9 and E10 thereon are discussed for simplicity and is understood to be representative of the plurality of spines and their electrodes of the basket assembly. As represented in the embodiment of FIG. 10, electrodes E1- E10 are disposed on the spine, arranged in pairs with electrodes in a respective pair being in closer proximity to each other than to electrodes of other pairs. As depicted in FIG. 11, FIG. 12 and FIG. 13, electrode pair E3, E4 are not in contact with tissue T, whereas electrode pairs E1, E2 and E9, E10 are in contact with tissue T. Accordingly, in some embodiments, the system controller commands the processing unit 441 to execute the force sensing module 468 to activate the force sensors FS1 - FS10. In response to the force sensing module 468 detecting the electrodes E1, E2, E9, E10 with tissue contact and the electrodes E3, E4 without contact (or without sufficient contact), the system commands the processing unit 441 to execute the switch control module 473 to control the switch circuitry 451 in setting each switch SW3 and SW4 for electrodes E3, E4 to the float setting S3 as shown in **FIG. 11****,** **FIG. 12** and **FIG. 13****.** The switch setting for the switches SW1, SW2, SW9, SW10 for remaining electrodes E1, E2, E9 and E10 in contact (or sufficient contact equal or greater a contact threshold) with tissue depends on the mode of operation selected by the user.

For sensing electrical activity of the heart tissue, the user activates a first control switch to select tissue sensing (e.g., for mapping). In response, the system controller commands the processing unit 441 to execute the tissue sensing subroutine 474 of the switch control module 473. As shown in the embodiment of **FIG. 11****,** the switch circuitry 451 accordingly sets switches SW1, SW2, SW9, SW10 for, respectively, the electrodes E1, E2, E9 and E10 to setting S2 in connecting each of these electrodes to the processing unit 41 as "return" electrodes for sensing tissue signals, including IEGM signals, from the heart tissue, wherein the system controller commands the processing unit 441 to execute the tissue sensing subroutine 474 to receive and process such signals into electrograms. The switch circuitry 451 also sets the switch SWW for electrode EREF to setting S3 in connecting the electrode to a float S6 as an "inactive" electrode. The path of electrical current from the heart tissue to the processing unit is shown in dotted lines.

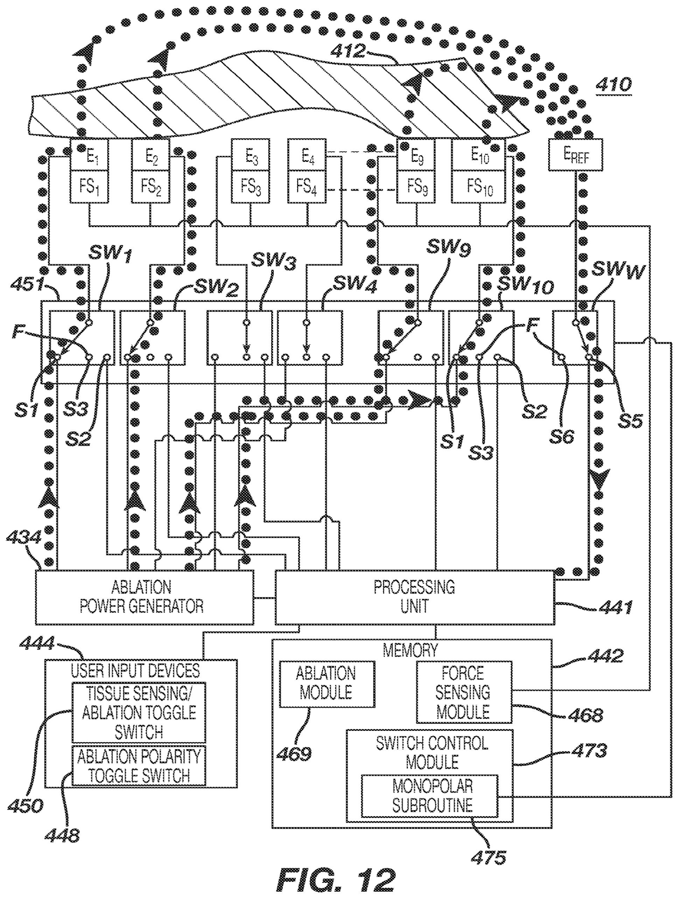

For ablating heart tissue, the user activates the first control switch to select ablation and a second control switch to select unipolarity or bipolarity. Where unipolar ablation is selected, the system controller commands the processing unit 441 to execute the unipolar subroutine 475 of the switch control module 473. As shown in the embodiment of **FIG. 12****,** the switch circuitry 451 accordingly sets each switch SW1, SW2, SW9, SW10 for, respectively, the electrodes E1, E2, E9, E10 to setting S1 in connecting these electrodes to the ablation power generator 434 as "active" for delivery ablation energy, including pulsed energy for IRE, and also sets the switch SWW for the electrode EREF to setting S5 connecting this electrode as a "return" electrode to the processing unit 441. The path of electrical current from the ablation power generator 434 to the processing unit 441 is shown in dotted lines, wherein a composite electrode is created from the "shorted" circuit of the electrodes E1, E2, E9, E10, that are in tissue contact and ablation power is withheld from the electrodes E3, E4 which are disconnected from the circuit. Thus, ablation is applied solely where the electrodes are in contact (or in sufficient contact equal to or greater than a threshold contact force) and the patient is exposed to only the necessary amount of ablation power.

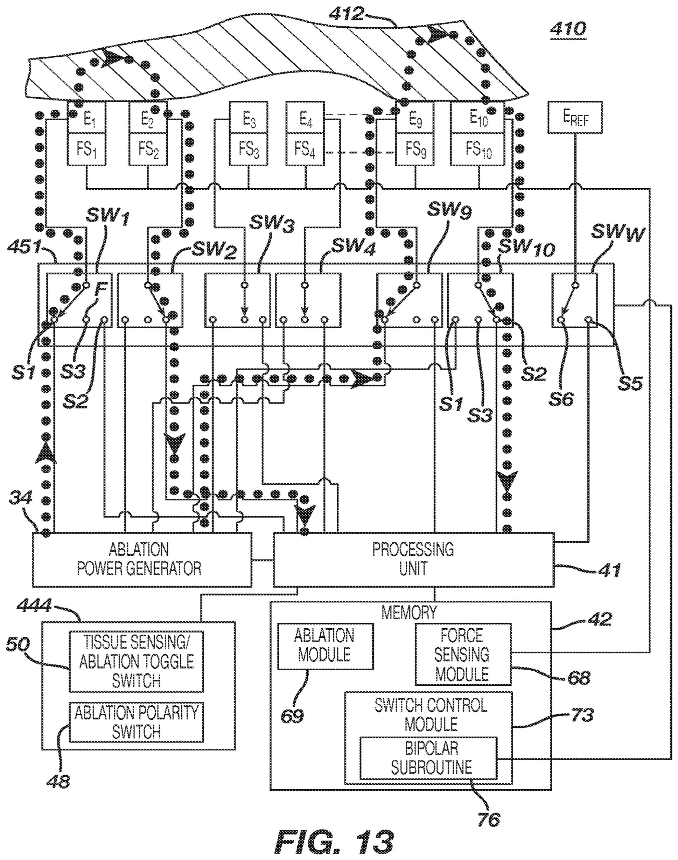

For ablating heart tissue in bipolarity, the user selects bipolar ablation using the second control switch. Accordingly, the system controller commands the processing unit 441 to execute the bipolar subroutine 476 of the switch control module 473. As shown in the embodiment of **FIG. 13****,** the switch circuitry 51 accordingly sets each switch SW1, SW10 for, respectively, the electrodes E1, E10 to setting S1 in connecting these electrodes to the ablation power generator 434 as 'active" electrodes for delivery ablation energy, including pulsed energy for IRE, while setting each switch SW2, SW9 for, respectively, the electrodes E2, E9 to setting S2 connecting these electrodes to the processing unit 441 as "return" electrodes to the processing unit 441. The switch circuitry 451 also sets the switch SWW for the electrode EREF to setting S6 connecting the electrode to a float as an "inactive" electrode. The path of electrical current from the ablation power generator 434 to the processing unit 441 is shown in dotted lines, where a composite electrode is created from the "shorted" circuit of the electrodes E1, E10.

In an IRE procedure, the pulsed frequency ablation (PFA) signals are delivered to "active" electrodes of either unipolar ablation or bipolar ablation having one or more pulsed trains ("pulse bursts") with pauses between the pulse trains. The pauses permit muscle relaxation if any contraction occurs as well as allowing the tissue to cool.

For the embodiment of the basket assembly of FIG. 9 when used in a tubular region such as a pulmonary vein, contact with tissue most often occurs around the equatorial region of the basket assembly that includes, for example, electrodes E5, E6, E7, E8 of selected or all of the spines of the basket assembly. Accordingly, the processing unit would actuate the switch circuitry to electrically connect those equatorial electrodes to create one or more composite electrode for ablating the pulmonary vein. Bipolar ablation may be accomplished with electrical energy from the ablation power generator traveling from, for example, the equatorial electrodes E5, E6, E7, E8 on spine 321A to equatorial electrodes E5, E6, E7, E8 on spine 321B or on spine 321C skipping over spine 321B, or even to the center ring electrode 340, depending on the electrical connections provided by the switch circuitry. Unipolar ablation may be accomplished with electrical energy from the ablation power generator traveling from the equatorial electrodes E5, E6, E7, E8 to body surface electrodes 38 on the patient's skin (see FIG.1) .

The preceding description has been presented with reference to presently preferred embodiments of the invention. Workers skilled in the art and technology to which this invention pertains will appreciate that alterations and changes in the described structure may be practiced without meaningfully departing from the principal, spirit and scope of this invention. Any feature or structure disclosed in one embodiment may be incorporated in lieu of or in addition to other features of any other embodiments, as needed or appropriate. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

It is noted that the electrodes described and illustrated herein are not limited to mapping (i.e., sensing signals or recording signals) but can be used to deliver energy such as RF (alternating cycle) or IRE (DC pulses) in bipolar or unipolar mode alone or in combination with the mapping or sensing function. In the application for IRE, and by way of example only, the electrodes may be configured to deliver at least 900V per electrode with a current of at least 10 amperes over a number of pulses sufficient to cause cell apoptosis. It is also noted that the mapping and ablation described herein may be applied to other tissue and organs beyond the heart.

It should be understood that any of the embodiments described herein may include various other features in addition to or in lieu of those described above. By way of example only, any of the embodiments described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A catheter system comprising:
a catheter including a basket assembly with a plurality of electrode to receive signals from cardiac tissue and a contact force sensor, the contact force sensor configured to generate first contact signals representative of a contact force acting on the basket assembly;
an ablation power generator configured to energize one or more of the plurality of electrodes to deliver energy into the cardiac tissue;
an impedance circuitry configured to send an electrical current to each electrode and generate second contact signals for each electrode representative of contact between the electrode and heart tissue;
a processor configured to receive the first and second contact signals and identify selected electrodes in contact with tissue and generate switch signals in response to the first and second contact signals; and
a switch circuitry configured to electrically connect in response to the switch signals solely the selected electrodes to the ablation power generator in forming a composite ablation electrode.

2. The catheter system of claim 1, wherein the switch circuitry is configured to electrically connect the selected electrodes for bipolar ablation, or for unipolar ablation.

3. The catheter system of claim 1, wherein the basket assembly includes a shaft defining a longitudinal axis, a plurality of elongated strips that converge at their distal and proximal ends, and an elongated pusher extending through the shaft and longitudinally movable relative to the shaft, the elongated pusher having a distal end connected to the distal ends of the strips such that proximal movement of the elongated pusher relative to the shaft along the longitudinal axis bows the strips outwardly in expanding the basket assembly.

4. The catheter system of claim 3, wherein the catheter includes a position sensor housed in the pusher.

5. The catheter system of claim 3, wherein distal movement of the elongated pusher relative to the shaft along the longitudinal axis after proximal movement collapses the basket assembly.

6. The catheter system of claim 1, wherein the contact force sensor is housed in the shaft proximally of a distal end of the shaft.

7. The catheter system of claim 1, wherein the contact force sensor includes a first end, a second end and a deformable spring therebetween.

8. The catheter system of claim 7, wherein the contact force sensor further includes a magnetic field transmitter housed in the first end and a magnetic field receiver housed in the second end, the magnetic field receiver configured to be responsive to the magnetic field transmitter in generating displacement signals indicative of a displacement in spatial relationship between the magnetic field transmitter and the magnetic field sensor, optionally wherein the displacement signals are indicative of a distance and an angular direction of the displacement.

9. A catheter system comprising:
a catheter with a plurality of electrodes and a plurality of force sensors, each force sensor configured to generate a respective contact signal representative of a contact force acting on a respective electrode;
an ablation power generator configured to energize the plurality of electrodes;
a processor configured to receive each respective contact signal and n in response thereto generate a respective switch signal for each electrode; and
a switch circuitry configured to electrically connect in response to each respective switch signal solely selected electrodes in contact with tissue to the ablation power generator in forming a composite electrode.

10. The catheter system of claim 9, further comprising a user input device configured to actuate the ablation power generator.

11. The catheter system of claim 9, wherein the processor is configured to actuate the ablation power generator in a biphasic mode.

12. The catheter system of claim 9, wherein the processor is configured to actuate the switch circuitry between a unipolar mode and a bipolar mode or between a tissue sensing mode and an ablation mode.

13. The catheter system of claim 9, wherein the processing unit is configured to actuate the switch circuitry in response to a control signal from the user input device.

14. The catheter system of claim 9, wherein the catheter further comprises a reference electrode, optionally wherein the reference electrode is configured to avoid tissue contact.

15. The catheter system of claim 11, wherein the switch circuitry includes a first switch configured to connect a respective electrode to the ablation power generator, a second switch configured to connect the respective electrode to the processing unit and a third switch as a float, optionally wherein the processing unit is configured to actuate the switch circuitry to connect the respective electrode to the ablation power generator via the first switch in response to the contact signal.
